# EUROPEAN PATENT APPLICATION

(11) **EP 3 730 600 A1**
(43) Date of publication of application: **28.10.2020**
(21) Application number: 18855155.0
(22) Date of filing: 19.12.2018
(51) Int. Cl.: C12M 1/107, C12M 1/00, C12P 3/00, D01F 9/127, D01F 9/133, C01B 32/182

(54) **BIOGAS PLANT**

(30) Priority: 19.12.2017 ES 201731431
(71) Applicant: Biela Pamies, Javier, 25001 Lleida (ES)
(72) Inventor: Biela Pamies, Javier, 25001 Lleida (ES)
(74) Representative: Pons
(86) International application number: PCT/ES2018/070817
(87) International publication number: WO 2019/122484

(57) **Abstract**

The invention relates to a biogas plant (1) of the type comprising, at least, one anaerobic digester (2) to obtain biogas from organic waste material (3), and which further comprises:
- at least one graphene synthesis reactor (4), and
- a biogas supply (5) as a source of carbon for said reactor (4), coming from the digester (2).

## Description

### OBJECT OF THE INVENTION

The present invention relates to a biogas plant that incorporates elements for producing graphene or diamond film coating from the generated biogas. Biogas plant is understood as any plant that has, at least, one anaerobic digester to obtain biogas from organic waste material (a by-product of the agri-food industry and/or organic waste, for example waste water purification or urban organic waste plants.

### BACKGROUND OF THE INVENTION

To process organic waste material, such as by-products of the agri-food industry and/or organic or urban waste or water purification, biogas generation plants are known that comprise, at least, one anaerobic digester in which this material decomposes in the absence of oxygen by action of bacteria and/or other microorganisms.

The product of this digestion are digestates, which are solid-liquid mixtures that are very rich in organic material and can be processed to be used as fertilisers, but mainly the most important product of digestion is biogas, the combustion of which constitutes energy valorisation of the original waste. Currently, this combustion is used to generate electric energy, for which these plants have electric and/or thermal energy generators or cogenerators.

In general, digestion is a complex process that comprises several stages that are carried out in different sections of the digester.

The limitation of the current configuration of biogas plants with this configuration lies in the fact that the configuration thereof only enables the use of biogas for generation or cogeneration.

### DESCRIPTION OF THE INVENTION

The biogas plant of the invention has a configuration that enables graphene to be obtained as a product, thus increasing the profitability of the plant.

The biogas plant of the invention is of the type comprising, at least, one anaerobic digester to obtain biogas from organic waste material (by-products of the agri-food industry and/or organic and urban waste, or from water purification plants, for example) and in accordance with the invention, it further, it comprises:
- at least one graphene synthesis reactor, and
- a biogas supply as a source of carbon for said reactor, coming from the digester (directly or indirectly, that is, without prior accumulation or with it).

In this way, the configuration of current biogas plants is revolutionised since they can be re-valorized with the production of graphene from said biogas. Moreover, given that the biogas reaches concentrations of up to 60-70% of methane CH₄, there is an affordable source of carbon with a high concentration of the same, and which also entails the recovery of a waste. That is, graphene is obtained, which is a high-tech material on which a significant industrial development will probably be based (for example which can be used for manufacturing graphene batteries, screens or cables) from slurry, cow manure, urban waste and/or waste from water purification.

In addition, taking into account that the current price of graphene is around 20-10€ per gram, and that a biogas plant of 1MW o another power, either lower or higher, can produce around 2 tonnes a year of graphene depending on the size of the plant and the production of biogas, the economic result enables the amortisation of the plant in short periods of time. The municipal waste collection tax can also be reduced for the population where the plant is implemented since the graphene produced or the diamond coating can be sold for use in cutting tools or other utensils. It must also be taken into account that the diamond coating can also be synthesised in the graphene synthesis reactor.

### BRIEF DESCRIPTION OF THE DRAWINGS

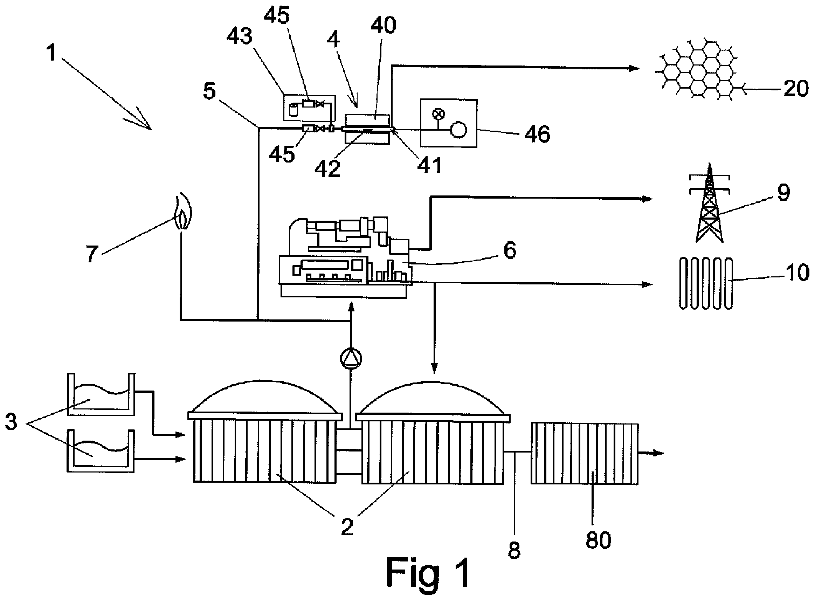

Figure 1 shows a schematic view of the biogas plant of the invention.

### DESCRIPTION OF A PRACTICAL EMBODIMENT OF THE INVENTION

The biogas plant (1) of the invention of the type comprising, at least. one anaerobic digester (2) to obtain biogas from organic waste material (3), and in accordance with the invention comprises:
- at least one graphene (20) synthesis reactor (4), and
- a biogas supply (5) as a source of carbon for said reactor (4), coming from the digester (2).

Ideally, the reactor (4) comprises a chemical vapour deposition (CVD) reactor since it is capable of producing very thin films of the material, which is an effect that is sought. Said reactor (4) comprises a tunnel oven (40) with a deposition chamber (41) for deposition on a substrate (42) (copper for example) and at, least, one gas source (43) (hydrogen and/or argon), as well as the source of carbon which constitutes the supply (5) of biogas.

The arrangement of flow controllers (45) in the biogas supply (5) and in the gas source (43) is preferred to be located before the inlet thereof into the reactor (4) in order to regulate the inlet flow thereof to control the synthesis of graphene or diamond film.

Furthermore, it has been envisaged that the reactor (4) comprises a low-pressure generator (46), which even reaches a vacuum, and which can also act as a by-product extractor since it improves the deposition of graphene on the substrate.

Similarly, the plant (1) can comprise, at least, one energy generator (6) combusting the biogas from the digester (2), such that the production of energy and/or graphene can be combined or alternated. The figure shows that the energy generator (6) is a cogenerator that produces electric energy (9) and thermal energy (10), part of which can be used to favour the operation of the digesters (2). The possible arrangement of an emergency torch (7) as a safety measure has also been envisaged.

Moreover, the plant comprises an additional outlet (8) for digestate, which can be used to obtain fertiliser, and which accumulates, for example, in the corresponding tank (80).

Having sufficiently described the nature of the invention, in addition to the practical embodiment, it is hereby stated that the arrangement indicated above and shown in the attached drawings may be changed in details provided that they do not change the fundamental principle.

## Claims

1. A biogas plant (1) of the type comprising at least one anaerobic digester (2) to obtain biogas from organic waste material (3) **characterised in that** it comprises:
- at least one graphene (20) synthesis reactor (4), and
- a biogas supply (5) as a source of carbon for said reactor (4), coming from the digester (2).

2. The biogas plant (1) according to claim 1, **characterised in that** the reactor (4) comprises a chemical vapour deposition reactor.

3. The biogas plant (1) according to any of the preceding claims, **characterised in that** the reactor (4) comprises a tunnel furnace (40) with a deposition chamber (41) for deposition on a substrate (42), at least a gas source (43), and the biogas supply (5).

4. The biogas plant (1) according to claim 3, **characterised in that** it comprises flow controls (45) arranged in the biogas supply (5) and in the gas source (43), prior to the inlet thereof into the reactor (4).

5. The biogas plant (1) according to any of the preceding claims, **characterised in that** the reactor (4) comprises a low-pressure generator (46).

6. The biogas plant (1) according to any of the preceding claims, **characterised in that** it comprises at least one energy generator (6) combusting the biogas from the digester (2).

7. The biogas plant (1) according to any of the preceding claims, **characterised in that** it comprises an emergency torch (7).

8. The biogas plant (1) according to any of the preceding claims, **characterised in that** it comprises an additional outlet (8) for digestate.
